(19) Europäisches Patentamt
European Patent Office
Office européen des brevets

(11) Veröffentlichungsnummer : **0 044 425**
**B1**

(12) # EUROPÄISCHE PATENTSCHRIFT

(45) Veröffentlichungstag der Patentschrift : **18.09.85**

(51) Int. Cl.⁴ : **C 07 D249/08**, A 01 N 43/64// C07C49/17, C07C49/16

(21) Anmeldenummer : **81104778.6**

(22) Anmeldetag : **22.06.81**

(54) **Halogenierte Triazolylvinyl-keto- und -carbinol-Derivate, Verfahren zu ihrer Herstellung sowie ihre Verwendung als Pflanzenwachstumsregulatoren und Fungizide.**

(30) Priorität : **03.07.80 DE 3025242**
**20.03.81 DE 3111012**

(43) Veröffentlichungstag der Anmeldung : **27.01.82 Patentblatt 82/04**

(45) Bekanntmachung des Hinweises auf die Patenterteilung : **18.09.85 Patentblatt 85/38**

(84) Benannte Vertragsstaaten : **AT BE CH DE FR GB IT LI NL SE**

(56) Entgegenhaltungen :
**EP-A- 0 020 955**
**EP-A- 0 022 975**
**EP-A- 0 032 239**
**DE-A- 2 838 847**
**DE-A- 3 010 560**
**US-A- 4 182 862**
**Die Akte enthält technische Angaben, die nach dem Eingang der Anmeldung eingereicht wurden und die nicht in dieser Patentschrift enthalten sind.**

(73) Patentinhaber : **BAYER AG**
**Konzernverwaltung RP Patentabteilung**
**D-5090 Leverkusen 1 Bayerwerk (DE)**

(72) Erfinder : **Regel, Erik**
**Bergerheide 72a**
**D-5600 Wuppertal 1 (DE)**
Erfinder : **Büchel, Karl Heinz, Prof. Dr.**
**Dabringhausenerstrasse 42**
**D-5093 Burcheid (DE)**
Erfinder : **Lürssen, Klaus, Dr.**
**August-Kierspel-Strasse 89**
**D-5060 Berg.-Gladbach 2 (DE)**
Erfinder : **Frohberger, Paul-Ernst, Dr.**
**Willi-Baumeisterstrasse 5**
**D-5090 Leverkusen (DE)**
Erfinder : **Brandes, Wilhelm, Dr.**
**Eichendorffstrasse 3**
**D-5653 Leichlingen (DE)**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



Jouve, 18, rue St-Denis, 75001 Paris, France

**Beschreibung**

Die Erfindung betrifft neue halogenierte Triazolylvinyl-keto- und -carbinol-Derivate, ein Verfahren zu ihrer Herstellung sowie ihre Verwendung als Pflanzenwachstumsregulatoren und Fungizide.

Es ist bereits bekannt geworden, daß bestimmte 4,4-Dimethyl-1-phenyl-2-triazolyl-penten-3-one und -ole eine gute fungizide Wirksamkeit besitzen (vgl. JP-A 53-130 661 und DE-A 28 38 847). Die Wirkung dieser Verbindungen ist jedoch, insbesondere bei niedrigen Aufwandmengen und -konzentrationen, nicht immer voll befriedigend. Die pflanzenwachstumsregulierende Wirkung dieser Azol-Derivate ist ebenfalls nicht immer ganz befriedigend.

Weiterhin werden in der DE-A 30 10 560 zahlreiche Azol-Derivate mit fungiziden und pflanzenwuchsregulierenden Eigenschaften offenbart. Keine der vorbeschriebenen Verbindungen enthält aber einen halogenierten tert.-Butyl-Rest, der über eine Keto- bzw. CH(OH)-Gruppe mit dem verbleibenden Molekülteil verbunden ist.

Im übrigen sind aus der EP-A 0 032 239 Triazolylvinylketo- und carbinol-Derivate mit fungizider Wirksamkeit bekannt. Allerdings werden in der betreffenden Druckschrift keine Stoffe erwähnt, in denen der über die Carbonyl-Gruppe gebundene Rest für Halo-tert.-Butyl steht und gleichzeitig der mit der Vinyl-Gruppe verknüpfte Rest (dort als $R^2$ bezeichnet) für gegebenenfalls substituiertes Phenyl steht.

Es wurden nun neue halogenierte Triazolylvinyl-keto- und -carbinol-Derivate der Formel (I)

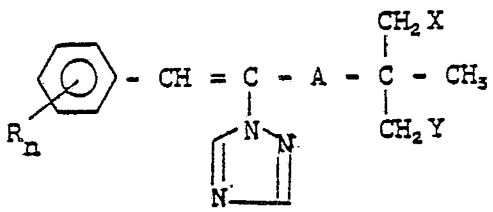

(I)

in welcher

A für die Ketogruppe oder die CH(OH)-Gruppierung steht,

X für Wasserstoff, Fluor, Chlor oder Brom steht,

Y für Fluor, Chlor oder Brom steht,

R für Fluor, Chlor, Brom, Alkyl mit 1 bis 4 Kohlenstoffatomen, Alkoxy mit 1 bis 4 Kohlenstoffatomen, Alkylthio mit 1 bis 4 Kohlenstoffatomen, Halogenalkyl mit 1 oder 2 Kohlenstoffatomen und bis zu 5 Halogenatomen, Halogenalkoxy mit 1 oder 2 Kohlenstoffatomen und bis zu 5 Halogenatomen, Halogenalkylthio mit 1 oder 2 Kohlenstoffatomen und bis zu 5 Halogenatomen, Alkylamino mit 1 oder 2 Kohlenstoffatomen, Dialkylamino mit 1 oder 2 Kohlenstoffatomen je Alkylgruppe, Nitro, Cyano, Hydroxy, Alkylcarbonyloxy mit 1 bis 4 Kohlenstoffatomen in der Alkylgruppe, für gegebenenfalls durch Fluor, Chlor, Brom und/oder Alkyl mit 1 oder 2 Kohlenstoffatomen substituiertes Phenyl, für gegebenenfalls durch Fluor, Chlor, Brom und/oder Alkyl mit 1 oder 2 Kohlenstoffatomen substituiertes Phenoxy oder für gegebenenfalls durch Fluor, Chlor, Brom und/oder Alkyl mit 1 oder 2 Kohlenstoffatomen substituiertes Benzyloxy steht und

n für die Zahlen 0, 1, 2 oder 3 steht, sowie deren physiologisch verträgliche Säureadditionssalze, und Metallsalzkomplexe gefunden.

Die erfindungsgemäßen Verbindungen der Formel (I) kommen in den geometrischen Isomeren E (trans) und Z (cis) vor. Bei der E,Z-Nomenklatur werden die an der Doppelbindung stehenden Substituenten nach der Cahn-Ingold-Prelog-Regel nach abnehmender Priorität eingeordnet. Stehen die bevorzugten Substituenten auf derselben Seite der Doppelbindung, liegt die Konfiguration Z (abgeleitet von « zusammen ») vor, stehen sie auf entgegengesetzter Seite, liegt die Konfiguration E (abgeleitet von « entgegen ») vor. Wenn A für die CH(OH)-Gruppierung steht, liegt ein asymmetrisches Kohlenstoffatom vor, so daß die Verbindungen der Formel (I) in diesem Fall außerdem in zwei optischen Isomerenformen anfallen.

Die Erfindung betrifft sowohl die einzelnen Isomeren als auch Isomerengemische.

Weiterhin wurde gefunden, daß man die halogenierten Triazolylvinyl-keto- und -carbinol-Derivate der Formel (I) sowie deren Säureadditionssalze und Metallsalzkomplexe erhält, wenn man Triazolyl-ketone der Formel (II)

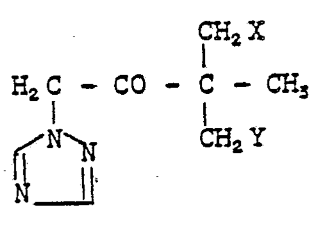

(II)

in welcher

X und Y die oben angegebene Bedeutung haben,

mit Aldehyden der Formel (III)

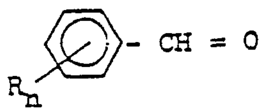

(III)

in welcher

R und n die oben angegebene Bedeutung haben,

in Gegenwart eines Lösungsmittels und in Gegenwart eines Katalysators umsetzt, gegebenenfalls anschließend die halogenierten Triazolylvinyl-keto-Derivate der Formel (Ia)

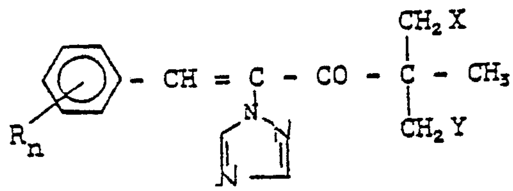

(Ia)

in welcher

X, Y, R und n die oben angegebene Bedeutung haben,

in allgemein üblicher Weise reduziert, und gegebenenfalls anschließend an die so erhaltenen Verbindungen der Formel (I) eine Säure oder ein Metallsalz addiert.

Schließlich wurde gefunden, daß die neuen halogenierten Triazolylvinyl-keto- und -carbinol-Derivate der Formel (I) sowie deren Säureadditionssalze und Metallsalzkomplexe starke pflanzenwachstumsregulierende und starke fungizide Eigenschaften aufweisen.

Überraschenderweise zeigen die erfindungsgemäßen Verbindungen der Formel (I) eine bessere fungizide und wachstumsregulierende Wirkung als die aus dem Stand der Technik bekannten 4,4-Dimethyl-1-phenyl-2-triazolyl-1-penten-3-one und -ole, welche chemisch und wirkungsmäßig naheliegende Verbindungen sind. So besitzen die erfindungsgemäßen Verbindungen beispielsweise deutlich bessere pflanzenwuchsregulierende und fungizide Eigenschaften als die aus der DE-A 28 38 847 bekannten Stoffe 1-Diphenyl-4,4-dimethyl-2-(1,2,4-triazol-1-yl)-pent-1-en-3-ol und 1-(2,4-Dichlorphenyl)-4,4-dimethyl-2-(1,2,4-triazol-1-yl)-pent-1-en-3-ol. Sie übertreffen ferner auch das in der EP-A 0 032 239 offenbarte 1-(4-Methoxyphenyl)-4,4-dimethyl-2-(1,2,4-triazol-1-yl)-pent-1-en-3-on bezüglich ihrer pflanzenwuchsregulierenden und fungiziden Aktivität.

Die erfindungsgemäßen Stoffe der Formel (I) sind dadurch charakterisiert, daß sie einen über Carbonyl oder Carbinol mit dem verbleibenden Molekülteil verbundenen Halotert.-Butyl-Rest enthalten und gleichzeitig einen gegebenenfalls substituierten Phenyl-Rest aufweisen, der über eine Vinylgruppe verknüpft ist. Da aus der DE-A 30 10 560 und der EP-A 0 032 239 keine Stoffe bekannt sind, die derartige Reste enthalten, unterscheiden sich die erfindungsgemäßen Verbindungen konstitutionell eindeutig von den ähnlichsten vorbeschriebenen Substanzen.

Besonders bevorzugt sind diejenigen Verbindungen der Formel (I), in denen X für Wasserstoff und Y für Fluor oder Chlor steht, oder X und Y gleich sind und für Fluor oder Chlor stehen ; R für Fluor, Chlor, Methyl, Isopropyl, tert.-Butyl, Methoxy, Methylthio, Isopropoxy, Trifluor-methyl, Difluorchlormethyl, Fluordichlormethyl, Trichlormethyl, 1,1,2-Trifluor-2-chlor-ethyl, Trifluormethoxy, Trifluormethylthio, 1,1,2-Trifluor-2-chlor-ethoxy und -ethylthio, Dimethylamino, Nitro, Cyano, Hydroxy, Acetoxy, tert.-Butylcarbonyloxy, sowie für gegebenenfalls einfach oder zweifach, gleich oder verschieden durch Fluor, Chlor und/oder Methyl substituiertes Phenyl, Phenoxy und Benzyloxy steht ; A und der Index n die in der Erfindungsdefinition angegebene Bedeutung haben.

Im einzelnen seien außer den bei den Herstellungsbeispielen genannten Verbindungen folgende Verbindungen der allgemeinen Formel (I) genannt :

Tabelle 1

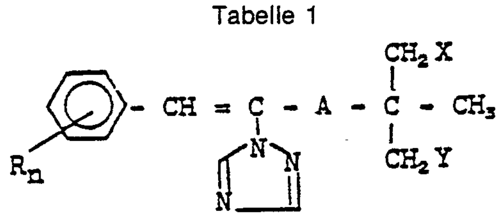

(I)

| $R_n$ | A | X | Y |
|---|---|---|---|
| 4-Cl | CO | Cl | Cl |
| 4-Cl | CH(OH) | Cl | Cl |
| $4-C(CH_3)_3$ | CO | Cl | Cl |

(Fortsetzung)

| $R_n$ | A | X | Y |
|---|---|---|---|
| $4-C(CH_3)_3$ | CH(OH) | Cl | Cl |
| $2,4-Cl_2$ | CO | Cl | Cl |
| $2,4-Cl_2$ | CH(OH) | Cl | Cl |
| 2-Cl | CO | Cl | Cl |
| 2-Cl | CH(OH) | Cl | Cl |
| 4-Cl | CO | F | F |
| 4-Cl | CH(OH) | F | F |
| $4-C(CH_3)_3$ | CO | F | F |
| $4-C(CH_3)_3$ | CH(OH) | F | F |
| $2,4-Cl_2$ | CO | F | F |
| $2,4-Cl_2$ | CH(OH) | F | F |
| 2-Cl | CO | F | F |
| 2-Cl | CH(OH) | F | F |
| $4-CF_3$ | CO | H | Cl |
| $2-CF_3$ | CO | H | Cl |
| 2-Cl, $4-CF_3$ | CO | H | Cl |
| $4-OCH_3$ | CO | H | Cl |
| $4-SCH_3$ | CO | H | Cl |
| 4-OH | CO | H | Cl |
| 2-OH;$3,5-Cl_2$ | CO | H | Cl |
| $4-NO_2$ | CO | H | Cl |
| 4-OH, $3-OCH_3$ | CO | H | Cl |
| 4-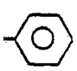 | CO | H | Cl |
| 4-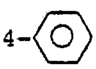-Cl | CO | H | Cl |
| 4-O-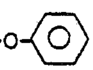 | CO | H | Cl |
| 4-O-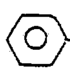-Cl | CO | H | Cl |
| $4-O-CH_2-$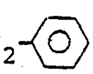 | CO | H | Cl |
| $4-O-CH_2-$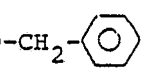-Cl | CO | H | Cl |
| $4-CF_3$ | CH(OH) | H | Cl |
| $2-CF_3$ | CH(OH) | H | Cl |
| 2-Cl, $4-CF_3$ | CH(OH) | H | Cl |
| $4-OCH_3$ | CH(OH) | H | Cl |
| $4-SCH_3$ | CH(OH) | H | Cl |
| 4-OH | CH(OH) | H | Cl |
| 2-OH;$3,5-Cl_2$ | CH(OH) | H | Cl |
| $4-NO_2$ | CH(OH) | H | Cl |
| 4-OH, $3-OCH_3$ | CH(OH) | H | Cl |

(Fortsetzung)

| $R_n$ | A | X | Y |
|---|---|---|---|
| 4-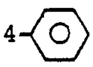 | CH(OH) | H | Cl |
| 4-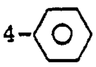-Cl | CH(OH) | H | Cl |
| 4-O-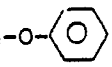 | CH(OH) | H | Cl |
| 4-O-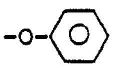-Cl | CH(OH) | H | Cl |
| 4-O-$CH_2$-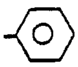 | CH(OH) | H | Cl |
| 4-O-$CH_2$-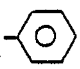-Cl | CH(OH) | H | Cl |
| 4-$CF_3$ | CO | H | F |
| 2-$CF_3$ | CO | H | F |
| 2-Cl, 4-$CF_3$ | CO | H | F |
| 4-$OCH_3$ | CO | H | F |
| 4-$SCH_3$ | CO | H | F |
| 4-OH | CO | H | F |
| 2-OH; 3,5-$Cl_2$ | CO | H | F |
| 4-$NO_2$ | CO | H | F |
| 4-OH, 3-$OCH_3$ | CO | H | F |
| 4-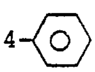 | CO | H | F |
| 4-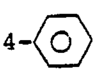-Cl | CO | H | F |
| 4-O-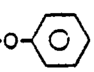 | CO | H | F |
| 4-O-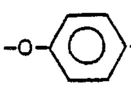-Cl | CO | H | F |
| 4-O-$CH_2$-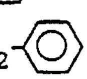 | CO | H | F |
| 4-O-$CH_2$-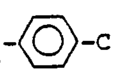-Cl | CO | H | F |
| 4-$CF_3$ | CH(OH) | H | F |
| 2-$CF_3$ | CH(OH) | H | F |
| 2-Cl, 4-$CF_3$ | CH(OH) | H | F |
| 4-$OCH_3$ | CH(OH) | H | F |
| 4-$SCH_3$ | CH(OH) | H | F |
| 4-OH | CH(OH) | H | F |
| 2-OH; 3,5-$Cl_2$ | CH(OH) | H | F |
| 4-$NO_2$ | CH(OH) | H | F |
| 4-OH, 4-$OCH_3$ | CH(OH) | H | F |

(Fortsetzung)

| $R_n$ | A | X | Y |
|---|---|---|---|
| 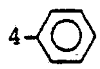 | CH(OH) | H | F |
| 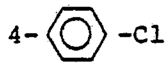 | CH(OH) | H | F |
| 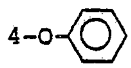 | CH(OH) | H | F |
| 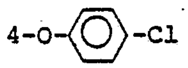 | CH(OH) | H | F |
| 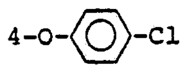 | CH(OH) | H | F |
| 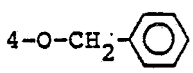 | CH(OH) | H | F |
| 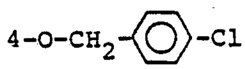 | CH(OH) | H | F |

Verwendet man beispielsweise Chlorpinakolyl-1,2,4-triazol und 4-Chlorbenzaldehyd als Ausgangsstoffe, so kann der Ablauf des erfindungsgemäßen Verfahrens durch das folgende Formelschema wiedergegeben werden :

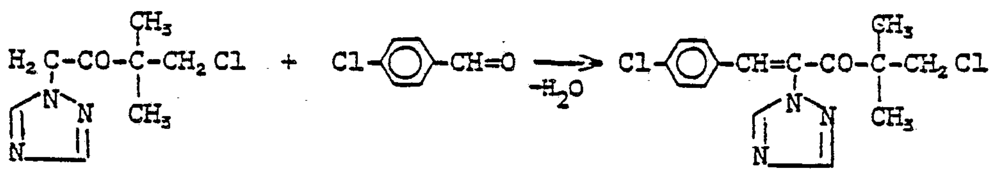


Verwendet man beispielsweise 1-Chlor-5-(4-chlorphenyl)-2,2-dimethyl-4-(1,2,4-triazol-1-yl)-4-penten-3-on und Natriumborhydrid als Ausgangsstoffe, so kann der Ablauf des erfindungsgemäßen Verfahrens durch das folgende Formelschema wiedergegeben werden :

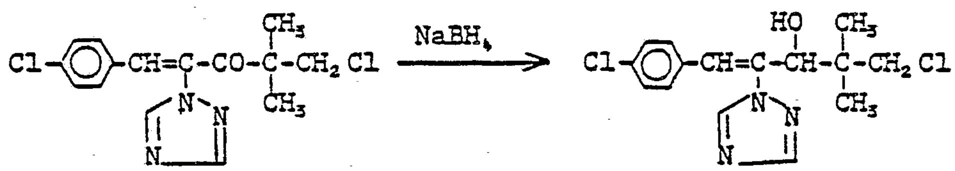


Die bei der Durchführung des erfindungsgemäßen Verfahrens als Ausgangsstoffe benötigten Triazolyl-ketone sind durch die Formel (II) allgemein definiert. In dieser Formel stehen X und Y für diejenigen Reste, die bereits im Zusammenhang mit der Beschreibung der erfindungsgemäßen Stoffe der Formel (I) für diese Substituenten genannt wurden.

Die Triazolyl-ketone der Formel (II) sind bekannt (vergleiche DE-A 2 820 361) oder können nach bekannten Verfahren synthetisiert werden. So erhält man Triazolylketone der Formel (II) zum Beispiel dadurch, daß man Halogenketone der Formel (IV)

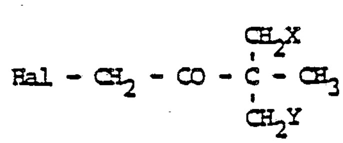 (IV)

in welcher

X und Y die oben angegebene Bedeutung haben und

Hal für Chlor oder Brom steht,

mit 1,2,4-Triazol in Gegenwart eines Verdünnungsmittels, wie beispielsweise Aceton, und in Gegenwart eines Säurebindemittels, wie beispielsweise Kaliumcarbonat, bei Temperaturen zwischen 20 und 150 °C umsetzt.

Die Halogenketone der Formel (IV) sind bekannt (vgl. z. B. DE-A 2 632 603 und DE-A 2 843 767) bzw. werden in allgemein bekannter Art und Weise erhalten, indem man Verbindungen der Formel (V)

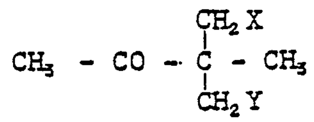

(V)

in welcher

X und Y die oben angegebene Bedeutung haben,

in Gegenwart eines inerten organischen Lösungsmittels bei Raumtemperatur mit Chlor oder Brom versetzt ; oder z. B. mit üblichen Chlorierungsmitteln, wie Sulfurylchlorid, bei 20 bis 60 °C umsetzt.

Die außerdem für das erfindungsgemäße Verfahren als Ausgangsstoffe zu verwendenden Aldehyde sind durch die Formel (III) allgemein definiert. In dieser Formel haben R und der Index n diejenigen Bedeutungen, die bereits im Zusammenhang mit der Beschreibung der erfindungsgemäße Stoffe (I) für diesen Substituenten bzw. für diesen Index genannt wurden.

Die Aldehyde der Formel (III) sind allgemein bekannte Verbindungen der organischen Chemie.

Als Lösungsmittel kommen für das erfindungsgemäße Verfahren vorzugsweise inerte organische Lösungsmittel in Frage. Hierzu gehören vorzugsweise Alkohole, wie Methanol und Ethanol ; Ether, wie Tetrahydrofuran und Dioxan ; aliphatische und cycloaliphatische Kohlenwasserstoffe, wie Hexan und Cyclohexan ; aromatische Kohlenwasserstoffe, wie Benzol, Toluol und Cumol ; halogenierte aliphatische und aromatische Kohlenwasserstoffe, wie Methylenchlorid, Tetrachlorkohlenstoff, Chloroform, Chlorbenzol und Dichlorbenzol.

Das erfindungsgemäße Verfahren wird in Gegenwart eines Katalysators durchgeführt. Man kann alle üblicherweise verwendbaren sauren und insbesondere basischen Katalysatoren sowie deren Puffergemische einsetzen. Hierzu gehören vorzugsweise Lewis-Säuren, wie z. B. Bortrifluorid, Bortrichlorid, Zinntetrachlorid oder Titantetrachlorid ; organische Basen wie Pyridin und Piperidin ; sowie insbesondere Piperidinacetat.

Die Reaktionstemperaturen können bei der Durchführung des erfindungsgemäßen Verfahrens in einem größeren Bereich variiert werden. Im allgemeinen arbeitet man zwischen 20 und 160 °C, vorzugsweise bei der Siedetemperatur des jeweiligen Lösungsmittels.

Bei der Durchführung des erfindungsgemäßen Verfahrens setzt man auf 1 Mol Triazolyl-keton der Formel (II) 1 bis 1,5 Mol Aldehyd der Formel (III) und katalytische bis 0,2 molare Mengen an Katalysator ein. Die Endprodukte der Formel (I) fallen vorzugsweise als E/Z-Isomerengemische an.

Eine Trennung in die reinen Isomeren ist auf übliche Arte und Weise möglich, wie z. B. durch Kristallisation oder durch chromatographische Trennverfahren.

Die erfindungsgemäße Reduktion erfolgt in üblicher Weise, z. B. durch Umsetzung mit komplexen Hydriden, gegebenenfalls in Gegenwart eines Verdünnungsmittels, oder durch Umsetzung mit Aluminiumisopropylat in Gegenwart eines Verdünnungsmittels.

Arbeitet man mit komplexen Hydriden, so kommen als Verdünnungsmittel für die erfindungsgemäße Umsetzung polare organische Lösungsmittel in Frage. Hierzu gehören vorzugsweise Alkohole, wie Methanol, Ethanol, Butanol, Isopropanol, und Ether, wie Diethylether oder Tetrahydrofuran. Die Reaktion wird im allgemeinen bei − 10 bis + 30 °C, vorzugsweise bei − 10 bis 20 °C durchgeführt. Hierzu setzt man auf 1 Mol des Ketons der Formel (Ia) etwa 1 Mol eines komplexen Hydrids, wie Natriumborhydrid, Calciumborhydrid oder Lithiumalanat, ein.

Zur Isolierung der reduzierten Verbindung der Formel (I) wird der Rückstand in verdünnter Salzsäure aufgenommen, anschließend alkalisch gestellt und mit einem organischen Lösungsmittel extrahiert. Die weitere Aufarbeitung erfolgt in üblicher Weise, ebenso eine eventuelle Trennung der E/Z-Isomerengemische.

Arbeitet man mit Aluminiumisopropylat, so kommen als Verdünnungsmittel für die erfindungsgemäße Umsetzung bevorzugt Alkohole, wie Isopropanol, oder inerte Kohlenwasserstoffe, wie Benzol, in Frage. Die Reaktionstemperaturen können wiederum in einem größeren Bereich variiert werden ; im allgemeinen arbeitet man zwischen 20 und 120 °C, vorzugsweise bei 50 bis 100 °C. Zur Durchführung der Reaktion setzt man auf 1 Mol des entsprechenden Ketons der Formel (Ia) etwa 1 bis 2 Mol Aluminiumisopropylat ein. Zur Isolierung der reduzierten Verbindungen der Formel (I) wird das überschüssige Lösungsmittel durch Destillation im Vakuum entfernt un die entstandene Aluminium-Verbindung mit verdünnter Schwefelsäure oder Natronlauge zersetzt. Die weitere Aufarbeitung erfolgt in üblicher Weise.

Bei der Reduktion mit Aluminiumisopropylat erhält man ausschließlich die Z-Isomeren.

Ein eindeutiges Charakterisierungsmerkmal für die beiden geometrischen Isomeren ist die $H^1$-Kernresonanz der zwei Triazol-Protonen. Die Differenz der Shiftwerte für diese beiden Protonen ist in der E-Formen ungefähr doppelt so groß wie in den entsprechenden Z-Formen.

Zur Herstellung von physiologisch verträglichen Säureadditionssalzen der Verbindungen der Formel (I) kommen vorzugsweise folgende Säuren in Frage : Die Halogenwasserstoffsäuren, wie z. B. Chlorwasserstoffsäure und die Bromwasserstoffsäure, insbesondere die Chlorwasserstoffsäure, ferner

Phosphorsäure, Salpetersäure, Schwefelsäure, mono- und bifunktionelle Carbonsäuren und Hydroxycarbonsäuren, wie z. B. Essigsäure, Maleinsäure, Bernsteinsäure, Fumarsäure, Weinsäure, sowie Sulfonsäuren, wie z. B. p-Toluolsulfonsäure und 1,5-Naphthalindisulfonsäure.

Die Säureadditionssalze der Verbindungen der Formel (I) können in einfacher Weise nach üblichen Salzbildungsmethoden, z. B. durch Lösen einer Verbindung der Formel (I) in einem geeigneten inerten Lösungsmittel und Hinzufügen der Säure, z. B. Chlorwasserstoffsäure, erhalten werden und in bekannter Weise, z. B. durch Abfiltrieren, isoliert und gegebenenfalls durch Waschen mit einem inerten organischen Lösungsmittel gereinigt werden.

Zur Herstellung von Metallsalz-Komplexen der Verbindungen der Formel (I) kommen vorzugsweise Salze von Metallen der II. bis IV. Hauptgruppe und der I. und II. sowie IV. bis VIII. Nebengruppe des Periodensystems in Frage, wobei Kupfer, Zink, Mangan, Magnesium, Zinn, Eisen und Nickel beispielhaft genannt seien.

Als Anionen der Salze kommen solche in Betracht, die sich vorzugsweise von folgenden Säuren ableiten : Halogenwasserstoffsäuren, wie z. B. Chlorwasserstoffsäure und Bromwasserstoffsäure, ferner Phosphorsäure und Schwefelsäure.

Die Metallsalz-Komplexe von Verbindungen der Formel (I) können in einfacher Weise nach üblichen Verfahren erhalten werden, so z. B. durch Lösen des Metallsalzes in Alkohol, z. B. Ethanol und Hinzufügen zur Verbindung der Formel (I). Man kann Metallsalz-Komplexe in bekannter Weise, z. B. Abfiltrieren, isolieren und gegebenenfalls durch Umkristallisation reinigen.

Die erfindungsgemäßen Wirkstoffe greifen in den Metabolismus der Pflanzen ein und können deshalb als Wachstumsregulatoren eingesetzt werden.

Für die Wirkungsweise von Pflanzenwachstumsregulatoren gilt nach der bisherigen Erfahrung, daß ein Wirkstoff auch mehrere verschiedenartige Wirkungen auf Pflanzen ausüben kann. Die Wirkungen der Stoffe hängen im wesentlichen ab von dem Zeitpunkt der Anwendung bezogen auf das Entwicklungsstadium der Pflanze sowie von den auf die Pflanzen oder ihre Umgebung ausgebrachten Wirkstoffmengen und von der Art der Applikation. In jedem Fall sollen Wachstumsregulatoren die Kulturpflanzen in bestimmter gewünschter Weise beeinflussen.

Pflanzenwuchsregulierende Stoffe können zum Beispiel zur Hemmung des vegetativen Wachstums der Pflanzen eingesetzt werden. Eine derartige Wuchshemmung ist unter anderem bei Gräsern von wirtschaftlichem Interesse, denn dadurch kann die Häufigkeit der Grasschnitte in Ziergärten, Park- und Sportanlagen, an Straßenrändern, auf Flughäfen oder in Obstanlagen reduziert werden. Von Bedeutung ist auch die Hemmung des Wuchses von krautigen und holzigen Pflanzen an Straßenrändern und in der Nähe von Pipelines oder Überlandleitungen oder ganz allgemein in Bereichen, in denen ein starker Zuwachs der Pflanzen unerwünscht ist.

Wichtig ist auch die Anwendung von Wachstumsregulatoren zur Hemmung des Längenwachstums von Getreide. Hierdurch wird die Gefahr des Umknickens (« Lagerns ») der Pflanzen vor der Ernte verringert oder vollkommen beseitigt. Außerdem können Wachstumsregulatoren bei Getreide eine Halmverstärkung hervorrufen, die ebenfalls dem Lagern entgegenwirkt. Die Anwendung von Wachstumsregulatoren zur Halmverkürzung und Halmverstärkung erlaubt es, höhere Düngermengen auszubringen, um den Ertrag zu steigern, ohne daß die Gefahr besteht, daß das Getreide lagert.

Eine Hemmung des vegetativen Wachstums ermöglicht bei vielen Kulturpflanzen eine dichtere Anpflanzung, sodaß Mehrerträge bezogen auf die Bodenfläche erzielt werden können. Ein Vorteil der so erzielten kleineren Pflanzen ist auch, daß die Kultur leichter bearbeitet und beerntet werden kann.

Eine Hemmung des vegetativen Wachstums der Pflanzen kann auch dadurch zu Ertragsteigerungen führen, daß die Nährstoffe und Assimilate in stärkerem Maße der Blüten- und Fruchtbildung zugute kommen als den vegetativen Pflanzenteilen.

Mit Wachstumsregulatoren läßt sich häufig auch eine Förderung des vegetativen Wachstums erzielen. Dies ist von großem Nutzen, wenn die vegetativen Pflanzenteile geerntet werden. Eine Förderung des vegetativen Wachstums kann aber auch gleichzeitig zu einer Förderung des generativen Wachstums führen, dadurch daß mehr Assimilate gebildet werden, sodaß mehr oder größere Früchte entstehen.

Ertragssteigerungen können in manchen Fällen durch einen Eingriff in den pflanzlichen Stoffwechsel erreicht werden, ohne daß sich Änderungen des vegetativen Wachstums bemerkbar machen. Ferner kann mit Wachstumsregulatoren eine Veränderung der Zusammensetzung der Pflanzen erreicht werden, was wiederum zu einer Qualitätsverbesserung der Ernteprodukte führen kann. So ist es beispielsweise möglich, den Gehalt an Zucker in Zuckerrüben, Zuckerrohr, Ananas sowie in Zitrusfrüchten zu erhöhen oder den Proteingehalt in Soja oder Getreide zu steigern. Auch ist es beispielsweise möglich, den Abbau erwünschter Inhaltsstoffe, wie z. B. Zucker in Zuckerrüben oder Zuckerrohr, mit Wachstumsregulatoren vor oder nach der Ernte zu hemmen. Außerdem läßt sich die Produktion oder der Abfluß von sekundären Pflanzeninhaltsstoffen positiv beeinflussen. Als Beispiel sei die Stimulierung des Latexflusses bei Gummibäumen genannt.

Unter dem Einfluß von Wachstumsregulatoren kann es zur Ausbildung parthenokarper Früchte kommen. Ferner kann das Geschlecht der Blüten beeinflußt werden. Auch kann eine Sterilität des Pollens erzeugt werden, was bei der Züchtung und Herstellung von Hybridsaatgut eine große Bedeutung hat.

Durch den Einsatz von Wachstumsregulatoren läßt sich die Verzweigung der Pflanzen steuern.

Einerseits kann durch Brechen der Apikaldominanz die Entwicklung von Seitentrieben gefördert werden, was besonders im Zierpflanzenbau auch in Verbindung mit einer Wuchshemmung sehr erwünscht sein kann. Andererseits ist es aber auch möglich, das Wachstum der Seitentriebe zu hemmen. Für diese Wirkung besteht z. B. großes Interesse im Tabakanbau oder bei der Anpflanzung von Tomaten.

Unter dem Einfluß von Wachstumsregulatoren kann der Blattbestand der Pflanzen so gesteuert werden, daß ein Entblättern der Pflanzen zu einem gewünschten Zeitpunkt erreicht wird. Eine derartige Entlaubung spielt bei der mechanischen Beerntung der Baumwolle eine große Rolle, ist aber auch in anderen Kulturen wie z. B. im Weinbau zur Erleichterung der Ernte von Interesse. Eine Entlaubung der Pflanzen kann auch vorgenommen werden, um die Transpiration der Pflanzen vor dem Verpflanzen herabzusetzen.

Ebenso läßt sich mit Wachstumsregulatoren der Fruchtfall steuern. Einerseits kann ein vorzeitiger Fruchtfall verhindert werden. Andererseits kann aber auch der Fruchtfall oder sogar das Abfallen der Blüten bis zu einem gewünschten Maße gefördert werden (« Ausdünnung »), um die Alternanz zu brechen. Unter Alternanz versteht man die Eigenart einiger Obstarten, endogen bedingt von Jahr zu Jahr sehr unterschiedliche Erträge zu bringen. Schließlich ist es möglich, mit Wachstumsregulatoren zum Zeitpunkt der Ernte die zum Ablösen der Früchte erforderlichen Kräfte zu reduzieren, um eine mechanische Beerntung zu ermöglichen oder eine manuelle Beerntung zu erleichtern.

Mit Wachstumsregulatoren läßt sich ferner eine Beschleunigung oder auch Verzögerung der Reife des Erntegutes vor oder nach der Ernte erreichen. Dieses ist von besonderem Vorteil, weil sich dadurch eine optimale Anpassung an die Bedürfnisse des Marktes herbeiführen läßt. Weiterhin können Wachstumsregulatoren in manchen Fällen die Fruchtausfärbung verbessern. Darüber hinaus kann mit Wachstumsregulatoren auch eine zeitliche Konzentrierung der Reife erzielt werden. Damit werden die Vorausetzungen dafür geschaffen, daß z. B. bei Tabak, Tomaten oder Kaffe eine vollständige mechanische oder manuelle Beerntung in einem Arbeitsgang vorgenommen werden kann.

Durch Anwendung von Wachstumsregulatoren kann ferner die Samen- oder Knospenruhe der Pflanzen beeinflußt werden, sodaß die Pflanzen, wie z. B. Ananas oder Zierpflanzen in Gärtnereien, zu einem Zeitpunkt keimen, austreiben oder blühen, an dem sie normalerweise hierzu keine Bereitschaft zeigen. Eine Verzögerung des Austriebes von Knospen oder der Keimung von Samen mit Hilfe von Wachstumsregulatoren kann in frostgefährdeten Gebieten erwünscht sein, um Schädigungen durch Spätfröste zu vermeiden.

Schließlich kann mit Wachstumsregulatoren eine Resistenz der Pflanzen gegen Frost, Trockenheit oder hohen Salzgehalt des Bodens induziert werden. Hierdurch wird die Kultivierung von Pflanzen in Gebieten möglich, die hierzu normalerweise ungeeignet sind.

Die erfindungsgemäßen Wirkstoffe weisen auch eine starke mikrobizide Wirkung auf und können zur Bekämpfung von unerwünschten Mikroorganismen praktisch eingesetzt werden. Die Wirkstoffe sind für den Gebrauch als Pflanzenschutzmittel geeignet.

Fungizide Mittel im Pflanzenschutz werden eingesetzt zur Bekämpfung von Plasmodiophoromycetes, Oomycetes, Chytridiomycetes, Zygomycetes, Ascomycetes, Basidiomycetes und Deuteromycetes.

Die gute Pflanzenverträglichkeit der Wirkstoffe in den zur Bekämpfung von Pflanzenkrankheiten notwendigen Konzentrationen erlaubt eine Behandlung von oberirdischen Pflanzenteilen, von Pflanz- und Saatgut, und des Bodens.

Als Pflanzenschutzmittel können die erfindungsgemäßen Wirkstoffe mit besonders gutem Erfolg zur Bekämpfung solcher Pilze eingesetzt werden, die echte Mehltauerkrankungen hervorrufen ; so zur Bekämpfung von Erysiphe-Arten, wie z. B. gegen den Erreger des Gersten- bzw. des Getreidemehltaus (Erysiphe graminis) und des Gurkenmehltaus (Erysiphe cichoracearum) ; sowie zur Bekämpfung von Pyricularia oryzae und Pellicularia sasakii.

Besonders hervorzuheben ist, daß die erfindungsgemäßen Wirkstoffe nicht nur eine protektive Wirkung entfalten, sondern auch systemisch wirksam sind. So gelingt es, Pflanzen gegen Pilzbefall zu schützen, wenn man den Wirkstoff über den Boden und die Wurzel oder über das Saatgut den oberirdischen Teilen der Pflanze zuführt. In entsprechenden Aufwandmengen zeigen die erfindungsgemäßen Verbindungen auch eine herbizide Wirkung.

Die Wirkstoffe können in die üblichen Formulierungen übergeführt werden, wie Lösungen, Emulsionen, Suspensionen, Pulver, Schäume, Pasten, Granulate, Aerosole, Wirkstoff-imprägnierte Natur- und synthetische Stoffe, Feinstverkapselungen in polymeren Stoffen und in Hüllmassen für Saatgut, ferner in Formulierung mit Brennsätzen, wie Räucherpatronen, -dosen, -spiralen u. ä., sowie ULV-Kalt- und Warmnebel-Formulierungen.

Diese Formulierungen werden in bekannter Weise hergestellt, z. B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z. B. auch organische Lösungsmittel als Hilfslösungsmittel verwendet werden. Als flüssige Lösungsmittel kommen im wesentlichen in Frage : Aromaten, wie Xylol, Toluol, oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z. B. Erdölfraktionen, Alkohole, wie Butanol oder Glycol, sowie deren Ether und Ester, Ketone,

wie Aceton, Methylethylketon, Methylisobutylketon oder Cyclohexanon, stark polare Lösungsmittel, wie Dimethylformamid und Dimethylsulfoxid, sowie Wasser; mit verflüssigten gasförmigen Streckmitteln oder Trägerstoffen sind solche Flüssigkeiten gemeint, welche bei normaler Temperatur und unter Normaldruck gasförmig sind, z. B. Aerosol-Treibgase, wie Halogenkohlenwasserstoffe sowie Butan, Propan, Stickstoff und Kohlendioxid; als feste Trägerstoffe kommen in Frage: z. B. natürliche Gesteinsmehle, wie Kaoline, Tonerden, Talkum, Kreide, Quarz, Attapulgit, Montmorillonit oder Diatomeenerde und synthetische Gesteinsmmehle, wie hochdisperse Kielselsäure, Aluminiumoxid und Silikate; als feste Trägerstoffe für Granulate kommen in Frage: z. B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel; als Emulgier- und/oder schaumerzeugende Mittel kommen in Frage: z. B. nichtionogene und anionische Emulgatoren, wie Polyoxyethylen-Fettsäure-Ester, Polyoxyethylen-Fettalkohol-ether, z. B. Alkylarylpolyglykol-ether, Alkylsulfonate, Alkylsulfate, Arylsulfonate sowie Eiweißhydrolysate; als Dispergiermittel kommen in Frage: z. B. Lignin-Sulfitablaugen und Methylcellulose.

Es können in den Formulierungen Haftmittel wie Carboxymethylcellulose, natürliche und synthetische pulverige, körnige und latexförmige Polymere verwendet werden, wie Gummiarabicum, Polyvinylalkohol, Polyvinylacetat.

Es können Farbstoffe wie anorganische Pigmente, z. B. Eisenoxid, Titanoxid, Ferrocyanblau und organische Farbstoffe, wie Alizarin-, Azo- und Metallphthalocyaninfarbstoffe und Spurennährstoffe, wie Salze von Eisen, Mangan, Bor, Kupfer, Kobalt, Molybdän und Zink verwendet werden.

Die Formulierungen enthalten im allgemeinen zwischen 0,1 und 95 Gew.-% Wirkstoff, vorzugsweise zwischen 0,5 und 90 %.

Die erfindungsgemäßen Wirkstoffe können in den Formulierungen oder in den verschiedenen Anwendungsformen in Mischung mit anderen bekannten Wirkstoffen vorliegen, wie Fungiziden, Bakteriziden, Insektiziden, Akariziden, Nematiziden, Herbiziden, Schutzstoffen gegen Vogelfraß, Wuchsstoffen, Pflanzennährstoffen, Bodenstrukturverbesserungsmitteln und Pflanzenwachstumsregulatoren.

Die Wirkstoffe können als solche, in Form ihrer Formulierungen oder der daraus durch weiteres Verdünnen bereiteten Anwendungsformen, wie gebrauchsfertige Lösungen, Emulsionen, Suspensionen, Pulver, Pasten und Granulate angewendet werden. Die Anwendung geschieht in üblicher Weise, z. B. durch Gießen, Tauchen, Spritzen, Sprühen, Vernebeln, Verdampfen, Injizieren, Verschlämmen, Verstreichen, Stäuben, Streuen, Trockenbeizen, Feuchtbeizen, Naßbeizen, Schlämmbeizen oder Inkrustieren. Es ist ferner möglich, die Wirkstoffe nach dem Ultra-Low-Volume-Verfahren auszubringen oder die Wirkstoffzubereitung oder den Wirkstoff selber in den Boden zu injizieren. Es kann auch das Saatgut der Pflanzen behandelt werden.

Beim Einsatz der erfindungsgemäßen Verbindungen als Pflanzenwachstumsregulatoren können die Aufwandmengen in einem größeren Bereich variiert werden. Im allgemeinen verwendet man pro Hektar Bodenfläche 0,01 bis 50 kg, bevorzugt 0,05 bis 10 kg an Wirkstoff.

Beim Einsatz der erfindungsgemäßen Stoffe als Pflanzenwachstumsregulatoren gilt, daß die Anwendung in einem bevorzugten Zeitraum vorgenommen wird, dessen genaue Abgrenzung sich nach den klimatischen und vegetativen Gegebenheiten richtet.

Auch beim Einsatz der erfindungsgemäßen Stoffe als Fungizide kann die Aufwandmenge je nach Art der Applikation in einem größeren Bereich variiert werden. So liegen die Wirkstoffkonzentrationen bei der Behandlung von Pflanzenteilen in den Anwendungsformen im allgemeinen zwischen 1 und 0,000 1 Gew.-%, vorzugsweise zwischen 0,5 und 0,001 Gew.-%. Bei der Saatgutbehandlung werden im allgemeinen Saatgut, vorzugsweise 0,01 bis 10 g, benötigt. Bei Behandlung des Bodens sind Wirkstoffkonzentrationen von 0,000 01 bis 0,1 Gew.-%, vorzugsweise von 0,000 1 bis 0,02 %, am Wirkungsort erforderlich.

## Herstellungsbeispiele

### Beispiel 1

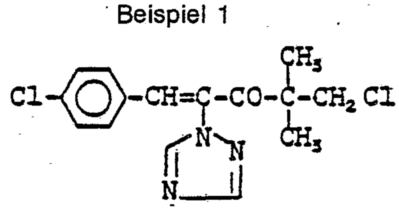

E/Z-Isomerengemisch

### Beispiel 2

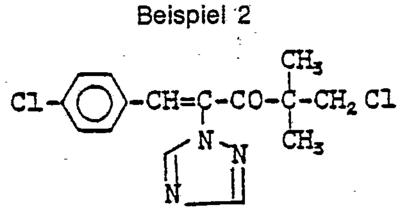

E-Isomeres

201,5 g (1 Mol) 1-Chlor-2,2-dimethyl-4-(1,2,4-triazol-1-yl)-butan-3-on, 140,5 g (1 Mol) 4-Chlorbenzylaldehyd sowie 9,9 ml Piperidin und 30 g Eisessig werden in 300 ml Toluol 8 Stunden unter Rückfluß erhitzt, wobei das entstandene Reaktionswasser azeotrop entfernt wird. Danach wird das Reaktionsgemisch mit Wasser und mit verdünnter Natriumhydrogencarbonatlösung gewaschen, über Natriumsulfat getrocknet und im Vakuum eingeengt. Man erhält 305,9 g (94,4 % der Theorie) rohes 1-Chlor-5-(4-chlorphenyl)-2,2-dimethyl-4-(1,2,4-triazol-1-yl)-4-penten-3-on vom Siedepunkt 165 °C/0,1 Torr als E,Z-Isomerengemisch. Durch Verrühren mit Isopropanol oder Ethanol läßt sich das E-Isomere in kristalliner Form isolieren. Es besitzt einen Schmelzpunkt von 112 °C.

Herstellung des Ausgangsproduktes

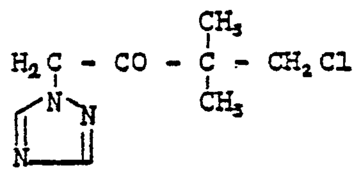

43,3 g (0,32 Mol) 1-Chlor-2,2-dimethyl-butan-2-on werden in 250 ml Ether gelöst und bei 20 °C unter Kühlung tropfenweise mit 52 g (0,325 Mol) Brom versetzt. Man läßt 1 Stunde nachrühren, wäscht die etherische Lösung fünfmal mit je 100 ml Wasser, trocknet über Natriumsulfat und engt im Vakuum ein. Das resultierende 4-Brom-1-chlor-2,2-dimethyl-butan-2-on wird in 50 ml Aceton aufgenommen und bei Raumtemperatur unter Kühlung tropfenweise zu einer Mischung aus 23,1 g (0,33 Mol) 1,2,4-Triazol und 46,2 g (0,4 Mol) Kaliumcarbonat in 250 ml Aceton gegeben. Man läßt 4 Stunden bei 20 °C nachrühren, saugt vom anorganischen Niederschlag ab und engt das Filtrat im Vakuum ein. Man erhält 57,9 g (90 % der Theorie) 1-Chlor-2,2-dimethyl-4-(1,2,4-triazol-1-yl)-butan-2-on, das direkt weiter umgesetzt werden kann.

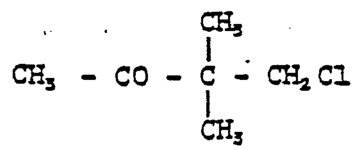

11,6 g (0,1 Mol) 2-Dimethyl-1-hydroxy-butan-3-on werden bei 50 bis 60 °C (Eiskühlung) zu 20,5 g (0,1 Mol) N,N-Diethyl-1,2,2-trichlorvinyl-amin getropft. Nach zweistündigem Rühren bei 60 °C wird im Wasserstrahlvakuum destilliert. Man erhält 8,1 g (60 % der Theorie) 1-Chlor-2,2-dimethyl-butan-3-on vom Schmelzpunkt 60-62 °C/12 mm Hg.

(Das 1-Chlor-2,2-dimethyl-butan-3-on wird in einer Ausbeute von 90 % erhalten, wenn man äquimolare Mengen 2,2-Dimethyl-1-hydroxy-butan-3-on und Triphenylphosphin in der zehnfachen Mengen Tetrachlorkohlenstoff 12 Stunden unter Rückfluß erhitzt, das Lösungsmittel abdestilliert, den Rückstand in Ether aufnimmt, filtriert und destilliert.)

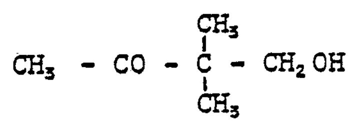

Zu 172 g (2 Mol) Methylisopropylketon in 1 000 ml Methanol werden 66 g (2,2 Mol) Paraformaldehyd und 1 g Kaliumhydroxid in 10 ml Methanol getropft. Man erhitzt 15 Stunden unter Rückfluß und destilliert anschließend das Methanol über eine Kolonne bei 82 °C Innentemperatur ab. Der Rückstand wird im Wasserstrahlvakuum destilliert. Man erhält 152,7 g (68 % der Theorie) 2,2-Dimethyl-1-hydroxy-butan-3-on vom Siedepunkt 80-82 °C/12 mm Hg.

Beispiel 3

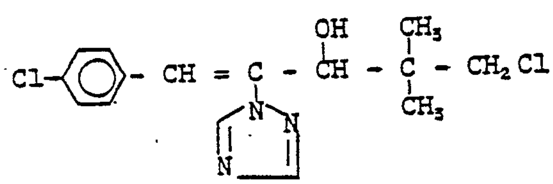

E/Z-Isomerengemisch

162 g (0,5 Mol) 1-Chlor-5-(4-chlorphenyl)-2,2-dimethyl-4-(1,2,4-triazol-1-yl)-4-penten-3-on (Beispiel 1) werden in 500 ml Isopropanol gelöst und unter Rühren portionsweise mit 9,5 g (0,25 Mol) Natriumborhydrid versetzt. Man läßt das Reaktionsgemisch 10 Stunden bei Raumtemperatur rühren und

engt anschließend im Vakuum ein. Der Rückstant wird in Toluol aufgenommen, mit verdünnter Essigsäure und anschließend mit Wasser gewaschen, über Natriumsulfat getrocknet und im Vakuum eingeengt. Man erhält 156,6 g (96 % der Theorie) 1-Chlor-5-(4-chlorphenyl)-2,2-dimethyl-4-(1,2,4-triazol-1-yl)-4-penten-3-ol vom Brechungsindex $n_D^{20} = 1{,}557\,9$ als E/Z-Isomerengemisch.

Beispiel 4

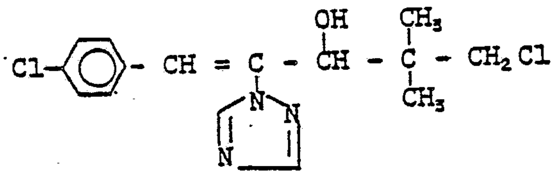

Z-Isomeres

48,6 g (0,15 Mol) 1-Chlor-5-(4-chlorphenyl)-2,2-dimethyl-4-(1,2,4-triazol-1-yl)-4-penten-3-on (Beispiel 1) und 30,6 g (0,15 Mol) Aluminiumisopropylat werden in 200 ml Isopropanol 7 Stunden unter Rückfluß erhitzt, wobei über eine 30 cm Vigreux-Kolonne kontinuierlich Isopropanol und Aceton abdestilliert bis im Destillat kein Aceton mehr nachzuweisen ist. Danach wird das Reaktionsgemisch eingeengt und der Rückstand mit Eis/Salzsäure versetzt. Man extrahiert mit Methylenchlorid. Die vereinigten Methylenchloridextrakte werden über Natriumsulfat getrocknet und im Vakuum eingeengt. Der ölige Rückstand wird säulenchromatographisch (Kieselgel/Chloroform) getrennt. Man erhält das nicht reduzierte E-Isomere des 1-Chlor-5-(4-chlorphenyl)-2,2-dimethyl-4-(1,2,4-triazol-1-yl)-penten-3-ons (Beispiel 2) sowie reines 1-Chlor-5-(4-chlorphenyl)-2,2-dimethyl-4-(1,2,4-triazol-1-yl)-penten-3-ol vom Schmelzpunkt 120 °C als Z-Isomeres.

Beispiel 5

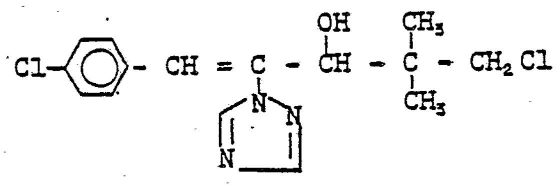

E-Isomeres

2,0 g (6,13 Mol) E-Isomeres des 1-Chlor-5-(4-chlorphenyl)-2,2-dimethyl-4-(1,2,4-triazol-1-yl)-4-penten-3-ons (Beispiel 2) und 0,467 g (4,11 m Mol) Calciumchlorid werden in 30 ml Isopropanol gelöst und bei − 5 °C tropfenweise mit einer Lösung von 0,167 g (4,3 m Mol) Natriumboprhydrid versetzt. Nach 6 Stunden wird das Reaktionsgemisch auf 25 °C erwärmt und im Vakuum eingeengt. Der Rückstand wird auf Wasser gegossen und mit Essigsäureethylester extrahiert. Die vereinigten Essigesterextrakte werden über Natriumsulfat getrocknet und im Vakuum eingeengt. Der ölige Rückstand kristallisiert beim Verrühren mit Diisopropylether. Man erhält 1,1 g (55 % der Theorie) 1-Chlor-5-(4-chlorphenyl)-2,2-dimethyl-4-(1,2,4-triazol-1-yl)-4-penten-3-ol vom Schmelzpunkt 170 °C als E-Isomeres.

In entsprechender Weise werden die folgenden Verbindungen der Formel (I) erhalten :

Tabelle 2

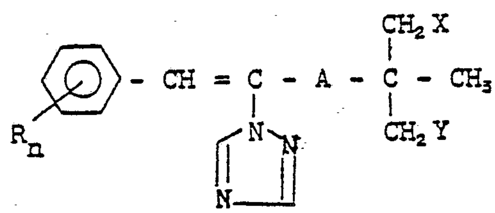

| Bsp. Nr. | $R_n$ | A | X | Y | Schmelzpunkt (°C) o. Brechungsindex $n_D^{20}$ |
|---|---|---|---|---|---|
| 6 | 4-Cl | CO | H | F | 1,5672 |
| 7 | $4\text{-}C(CH_3)_3$ | CO | H | F | 1,5565 |
| 8 | $4\text{-}C(CH_3)_3$ | CO | H | Cl | 1,5655 (Z-Isom.) |

(Fortsetzung)

| Bsp. Nr. | $R_n$ | A | X | Y | Schmelzpunkt (°C) o. Brechungsindex $n_D^{20}$ |
|---|---|---|---|---|---|
| 9 | 4-$N(CH_3)_2$ | CO | H | F | 100 |
| 10 | 4-$N(CH_3)_2$ | CO | H | Cl | 84 |
| 11 | 2-Cl | CO | H | F | 1,5632 (E-Isom.) |
| 12 | 4-Cl | CH(OH) | H | F | 230 (x NDS) |
| 13 | 4-Cl | CH(OH) | H | F | 110 (Z-Isom.) |
| 14 | 4-$C(CH_3)_3$ | CH(OH) | H | F | 1,5250 |
| 15 | 4-$C(CH_3)_3$ | CH(OH) | H | Cl | 1,5820 (Z-Isom.) |
| 16 | 4-$N(CH_3)_2$ | CH(OH) | H | F | 118 (Z-Isom.) |
| 17 | 4-$N(CH_3)_2$ | CH(OH) | H | Cl | 120 (Z-Isom.) |
| 18 | 2-Cl | CH(OH) | H | F | 116 (Z-Isom.) |
| 19 | 2-Cl | CH(OH) | H | F | 140 (E-Isom.) |
| 20 | 4-$OCF_3$ | CH(OH) | H | Cl | 100 (Z-Isom.) |
| 21 | 4-$OCF_3$ | CH(OH) | H | Cl | 140 (E-Isom.) |
| 22 | 2,4-$Cl_2$ | CH(OH | H | F | 150 (Z-Isom.) |
| 23 | 2,4-$Cl_2$ | CH(OH) | H | F | 158 (E-Isom.) |
| 24 | 4-Cl | CO | F | F | 1,5728 |
| 25 | 2,4-$Cl_2$ | CO | F | F | 1,5778 |
| 26 | 4-Cl | CO | Cl | Cl | 1,5942 |
| 27 | 2,4-$Cl_2$ | CO | Cl | Cl | 1,5868 |
| 28 | 4-Cl | CH(OH) | F | F | 74 (E-Isom.) |
| 29 | 4-Cl | CH(OH) | F | F | 110 (Z-Isom.) |
| 30 | 2,4-$Cl_2$ | CH(OH) | F | F | 157 (E-Isom.) |
| 31 | 2,4-$Cl_2$ | CH(OH) | F | F | 154 (Z-Isom.) |
| 32 | 4-Cl | CH(OH) | Cl | Cl | 150 (Z-Isom.) |
| 33 | 2,4-$Cl_2$ | CH(OH) | Cl | Cl | 148 (Z-Isom.) |
| 34 | 4-Cl | CH(OH) | H | F | 88 (Z-Isom.) |
| 35 | 4-F | CH(OH) | H | Cl | 122 (Z-Isom.) |
| 36 | 4-F | CH(OH) | H | Cl | 150 (E-Isom.) |
| 37 | 4-F | CH(OH) | H | F | 108 (Z-Isom.) |
| 38 | 4-Cl | CH(OH) | H | F | 1,5421 |
| 39 | 2,4-$Cl_2$ | CH(OH) | H | Cl | 150 (Z-Isom.) |
| 40 | 2,4-$Cl_2$ | CH(OH) | H | Cl | 140 (E-Isom.) |
| 41 | 4-F | CO | H | Cl | 58 (E-Isom.) |
| 42 | 2,4-$Cl_2$ | CO | H | F | Öl (E-Isom.) |

NDS = 1,5-Naphthalindisulfonsäure

## Anwendungsbeispiele

In den nachfolgenden Anwendungsbeispielen werden die nachstehend angegebenen Verbindungen als Vergleichssubstanzen eingesetzt :

A = 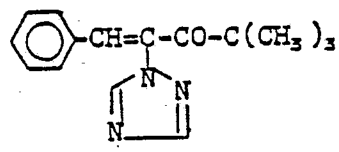

B = 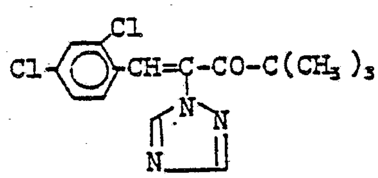

C = 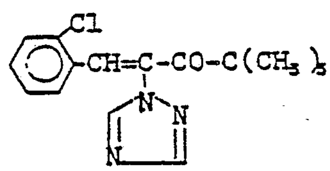

D = 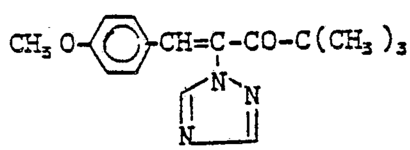 (bekannt aus EP-A 0 032 239)

E = 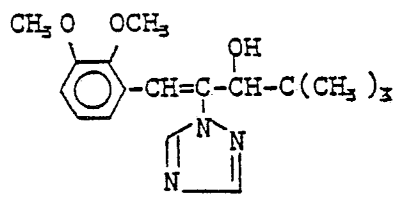

F = 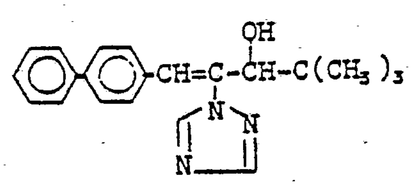 (bekannt aus DE-A 28 38 847)

G = 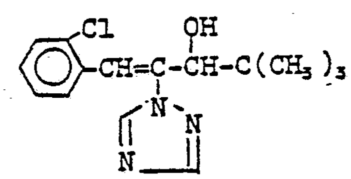

H = 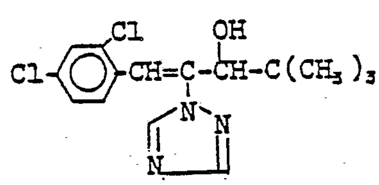 (bekannt aus DE-A 28 38 847)

Beispiel A

Wuchshemmung bei Sojabohnen

Lösungsmittel : 30 Gewichtsteile Dimethylformamid
Emulgator : 1 Gewichtsteil Polyoxyetylen-Sorbitan-Monolaurat

Zur Herstellung einer zweckmäßigen Wirkstoffzubereitung vermischt man 1 Gewichtsteil Wirkstoff mit den angegebenen Mengen Lösungsmittel und Emulgator und füllt mit Wasser auf die gewünschte Konzentration auf.

Sojabohnenpflanzen werden im Gewächshaus bis zur vollen Entfaltung des ersten Folgeblattes angezogen. In diesem Stadium werden die Pflanzen tropfnaß mit den Wirkstoffzubereitungen besprüht. Nach 3 Wochen wird bei allen Pflanzen der Zuwachs gemessen und die Wuchshemmung in Prozent des Zuwachses der Kontrollpflanzen berechnet. Es bedeuten 100 % Wuchshemmung den Stillstand des Wachstums und 0 % ein Wachstum entsprechend dem der Kontrollpflanzen.

Eine sehr deutliche Überlegenheit in der Wirksamkeit gegenüber den aus dem Stand der Technik bekannten Verbindungen (A), (B), (D), (E), (F) und (G) zeigen bei diesem Test z. B. die Verbindungen gemäß folgenden Herstellungsbeispielen : 3, 4, 7, 12, 13, 14, 35, 36, 37, 38 und 40.

Beispiel B

Wuchshemmung bei Baumwolle

Lösungsmittel : 30 Gewichtsteile Dimethylformamid
Emulgator : 1 Gewichtsteil Polyoxyethylen-Sorbitan-Monolaurat

Zur Herstellung einer zweckmäßigen Wirkstoffzubereitung vermischt man 1 Gewichtsteil Wirkstoff mit den angegebenen Mengen Lösungsmittel und Emulgator und füllt mit Wasser auf die gewünschte Konzentration auf.

Baumwollpflanzen werden im Gewächshaus bis zur vollen Entfaltung des 5. Folgeblattes angezogen. In diesem Stadium werden die Pflanzen tropfnaß mit den Wirkstoffzubereitungen besprüht. Nach 3 Wochen wird der Zuwachs der Pflanzen gemessen und die Wuchshemmung in Prozent des Zuwachses der Kontrollpflanzen berechnet. Es bedeuten 100 % Wuchshemmung den Stillstand des Wachstums und 0 % ein Wachstum entsprechend dem der Kontrollpflanzen.

Eine sehr deutliche Überlegenheit in der Wirksamkeit gegenüber den aus dem Stand der Technik bekannten Verbindungen (A), (B), (E), (F) und (H) zeigen bei diesem Test. z. B. die Verbindungen gemäß folgenden Herstellungsbeispielen : 3, 4, 7, 13, 14, 36, 37, 38 und 40.

Beispiel C

Wuchshemmung bei Zuckerrüben

Lösungsmittel : 30 Gewichtsteile Dimethylformamid
Emulgator : 1 Gewichtsteil Polyoxyethylen-Sorbitan-Monolaurat

Zur Herstellung einer zweckmäßigen Wirkstoffzubereitung vermischt man 1 Gewichtsteil Wirkstoff mit den angegebenen Mengen Lösungsmittel und Emulgator und füllt mit Wasser auf die gewünschte Konzentration auf.

Zuckerrüben werden im Gewächshaus bis zur vollen Ausbildung der Keimblätter angezogen. In diesem Stadium werden die Pflanzen tropfnaß mit den Wirkstoffzubereitungen besprüht. Nach 14 Tagen wird der Zuwachs der Pflanzen gemessen und die Wuchshemmung in Prozent des Zuwachses der Kontrollpflanzen berechnet. Es bedeuten 0 % Wuchshemmung ein Wachstum entsprechend dem der Kontrollpflanzen. 100 % Wuchshemmung bedeutet Stillstand des Wachstums.

Die erfindungsgemäßen Wirkstoffe 3, 4, 12, 13, 35, 36, 37, 38, 39 und 40 zeigen in diesem Test eine bessere Wuchshemmung als die aus dem Stand der Technik bekannten Verbindungen (E) und (F).

Beispiel D

Trockenresistenz bei Zuckerrüben

Bei Behandlung junger Zuckerrübenpflanzen mit der Wirkstoffzubereitung wurde besonders bei dem erfindungsgemäßen Beispiel 5 in den Konzentrationen 250 und 125 ppm eine Resistenz gegenüber Boden-Trockenheit festgestellt.

Beispiel E

Erysiphe-Test (Gerste)/protektiv

Lösungsmittel : 100 Gewichtsteile Dimethylformamid
Emulgator : 0,25 Gewichtsteile Alkylaryl-polyglykolether

Zur Herstellung einer zweckmäßigen Wirkstoffzubereitung vermischt man 1 Gewichtsteil Wirkstoff

mit den angegebenen Mengen Lösungsmittel und Emulgator und verdünnt das Konzentrat mit Wasser auf die gewünschte Konzentration.

Zur Prüfung auf protektive Wirksamkeit besprüht man junge Pflanzen mit der Wirkstoffzubereitung taufeucht. Nach Antrocknen des Spritzbelages werden die Pflanzen mit Sporen von Erysiphe graminis f. sp. hordei bestäubt.

Die Pflanzen werden in einem Gewächshaus bei einer Temperatur von ca. 20 °C und einer relativen Luftfeuchtigkeit von ca. 80 % aufgestellt, um die Entwicklung von Mehltaupusteln zu begünstigen.

7 Tage nach der Inokulation erfolgt die Auswertung.

Eine deutliche Überlegenheit in der Wirksamkeit gegenüber dem Stand der Technik bekannten Verbindungen (B) und (E) zeigen bei diesem Test. z. B. die Verbindungen gemäß folgender Herstellungsbeispiele : 6, 7, 3, 4, 12, 13 und 14.

Beispiel F

Gerstenmehltau-Test (Erysiphe graminis var. hordei)/systemisch (pilzliche Getreidesproßkrankheit)

Die Anwendung der Wirkstoffe erfolgt als pulverförmige Saatgutbehandlungsmittel. Sie werden hergestellt durch Abstrecken des Wirkstoffes mit einem Gemisch aus gleichen Gewichtsteilen Talkum und Kieselgur zu einer feinpulverigen Mischung mit der gewünschten Wirkstoffkonzentration.

Zur Saatgutbehandlung schüttet man Gerstensaatgut mit dem abgestreckten Wirkstoff in einer verschlossenen Glasflasche. Das Saatgut sät man mit 3 × 12 Korn in Blumentöpfe 2 cm tief in ein Gemisch aus einem Volumenteil Fushstorfer Einheitserde und einem Volumenteil Quarzsand ein. Die Keimung und der Auflauf erfolgen unter günstigen Bedingungen im Gewächshaus. 7 Tage nach der Aussaat, wenn die Gerstenpflanzen ihr erstes Blatt entfaltet haben, wenden sie mit frischen Sporen von Erysiphe graminis var. hordei bestäubt und bei 21-22 °C und 80-90 % rel. Luftfeuchte und 16 stündiger Belichtung weiter kultiviert. Innerhalb von 6 Tagen bildet sich an den Blättern die typischen Mehltaupusteln aus.

Der Befallsgrad wird in Prozent des Befalls der unbehandelten Kontrollpflanzen ausgedrückt. So bedeutet 0 % keinen Befall und 100 % den gleichen Befallsgrad wie bei der unbehandelten Kontrolle. Der Wirkstoff ist umso wirksamer je geringer der Mehltaubefall ist.

Eine deutliche überlegenheit in der Wirksamkeit gegenüber der aus dem Stand der Technik bekannten Verbindung (H) zeigt bei diesem Test z. B. die Verbindung gemäß Herstellungsbeispiel 3.

Beispiel G

Erysiphe-Test (Gurken)/protektiv

Lösungsmittel : 4,7 Gew.-Teile Aceton
Emulgator : 0,3 Gew.-Teile Alkylaryl-polyglykolether
Wasser : 95,0 Gew.-Teile

Man vermischt die für die gewünschte Wirkstoffkonzentration in der Spritzflüssigkeit nötige Wirkstoffmenge mit der angegebenen Menge des Lösungsmittels und verdünnt das Konzentrat mit der angegebenen Menge Wasser, welches die genannten Zusätze enthält.

Mit der Spritzflüssigkeit bespritzt man junge Gurkenpflanzen mit etwa drei Laubblättern bis zur Tropfnässe. Die Gurkenpflanzen verbleiben zur Trocknung 24 Stunden im Gewächshaus. Dann werden sie zur Inokulation mit Konidien des Pilzes Erysiphe cichoracearum bestäubt. Die Pflanzen werden anschließend bei 23 bis 24 °C und bei einer relativen Luftfeuchtigkeit von ca. 75 % im Gewächshaus aufgestellt.

Nach 12 Tagen wird der Befall der Gurkenpflanzen bestimmt. Die erhaltenen Boniturwerte werden in Prozent Befall umgerechnet. 0 % bedeutet keinen Befall, 100 % bedeutet, daß die Pflanzen vollständig befallen sind.

Bei diesem Test zeigen z. B. die folgenden Verbindungen eine sehr gute Wirkung, die derjenigen der aus dem Stand der Technik bekannten Verbindungen (D) und (E) überlegen ist :

Verbindungen gemäß Herstellungsbeispielen : 6, 7, 14, 2 und 13.

**Patentansprüche**

1. Halogenierte Triazolylvinyl-keto- und -carbinol-Derivate der Formel (I)

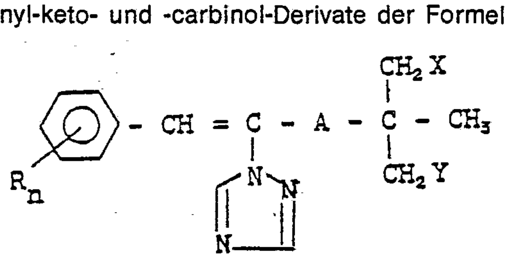

(I)

in welcher

A für die Ketogruppe oder die CH(OH)-Gruppierung steht,

X für Wasserstoff, Fluor, Chlor oder Brom steht,

Y für Fluor, Chlor oder Brom steht,

R für Fluor, Chlor, Brom, Alkyl mit 1 bis 4 Kohlenstoffatomen, Alkoxy mit 1 bis 4 Kohlenstoffatomen, Alkylthio mit 1 bis 4 Kohlenstoffatomen, Halogenalkyl mit 1 oder 2 Kohlenstoffatomen und bis zu 5 Halogenatomen, Halogenalkoxy mit 1 oder 2 Kohlenstoffatomen und bis zu 5 Halogenatomen, Halogenalkylthio mit 1 oder 2 Kohlenstoffatomen und bis zu 5 Halogenatomen, Alkylamino mit 1 oder 2 Kohlenstoffatomen, Dialkylamino mit 1 oder 2 Kohlenstoffatomen je Alkylgruppe, Nitro, Cyano, Hydroxy, Alkylcarbonyloxy mit 1 bis 4 Kohlenstoffatomen in der Alkylgruppe, für gegebenenfalls durch Fluor, Chlor, Brom und/oder Alkyl mit 1 oder 2 Kohlenstoffatomen substituiertes Phenyl, für gegebenenfalls durch Fluor, Chlor, Brom und/oder Alkyl mit 1 oder 2 Kohlenstoffatomen substituiertes Phenoxy oder für gegebenenfalls durch Fluor, Chlor, Brom und/oder Alkyl mit 1 oder 2 Kohlenstoffatomen substituiertes Benzyloxy steht und

n für die Zahlen 0, 1, 2 oder 3 steht, sowie deren physiologisch verträgliche Säure-Additionssalze und Metallsalzkomplexe.

2. Halogenierte Triazolylvinyl-keto- und -carbinol-Derivate der Formel (I), in denen X für Wasserstoff und Y für Fluor oder Chlor steht.

3. Halogenierte Triazolylvinyl-keto- und -carbinol-Derivate der Formel (I), in denen X und Y gleich sind und für Fluor oder Chlor stehen.

4. Halogenierte Triazolylvinyl-keto- und carbinol-Derivate der Formel (I), in denen R für Fluor, Chlor, Methyl, Isopropyl, tert.-Butyl, Methoxy, Methylthio, Isopropoxy, Trifluormethyl, Difluormethyl, Fluordichlormethyl, Trichlormethyl, 1,1,2-Trifluor-2-chlor-ethyl, Trifluormethoxy, Trifluormethylthio, 1,1,2-Trifluor-2-chlor-ethoxy und -ethylthio, Dimethylamino, Nitro, Cyano, Hydroxy, Acetoxy, tert.-Butylcarbonyloxy, sowie für gegebenenfalls einfach oder zweifach, gleich oder verschieden durch Fluor, Chlor und/oder Methyl substituiertes Phenyl, Phenoxy und Benzyloxy steht.

5. Verfahren zur Herstellung von halogenierten Triazolylvinyl-keto- und carbinol-Derivaten der Formel (I)

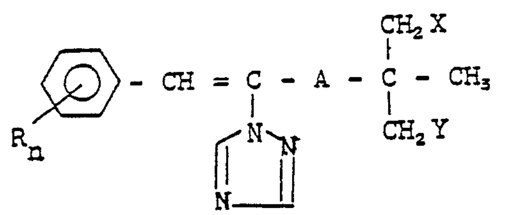

(I)

in welcher

A für die Ketogruppe oder die CH(OH)-Gruppierung steht,

X für Wasserstoff, Fluor, Chlor oder Brom steht,

Y für Fluor, Chlor oder Brom steht,

R für Fluor, Chlor, Brom, Alkyl mit 1 bis 4 Kohlenstoffatomen, Alkoxy mit 1 bis 4 Kohlenstoffatomen, Alkylthio mit 1 bis 4 Kohlenstoffatomen, Halogenalkyl mit 1 oder 2 Kohlenstoffatomen und bis zu 5 Halogenatomen, Halogenalkoxy mit 1 oder 2 Kohlenstoffatomen und bis zu 5 Halogenatomen, Halogenalkylthio mit 1 oder 2 Kohlenstoffatomen und bis zu 5 Halogenatomen, Alkylamino mit 1 oder 2 Kohlenstoffatomen, Dialkylamino mit 1 oder 2 Kohlenstoffatomen je Alkylgruppe, Nitro, Cyano, Hydroxy, Alkylcarbonyloxy mit 1 bis 4 Kohlenstoffatomen in der Alkylgruppe, für gegebenenfalls durch Fluor, Chlor, Brom und/oder Alkyl mit 1 oder 2 Kohlenstoffatomen substituiertes Phenyl, für gegebenenfalls durch Fluor, Chlor, Brom und/oder Alkyl mit 1 oder 2 Kohlenstoffatomen substituiertes Phenoxy oder für gegebenenfalls durch Fluor, Chlor, Brom und/oder Alkyl mit 1 oder 2 Kohlenstoffatomen substituiertes Benzyloxy steht und

n für die Zahlen 0, 1, 2 oder 3 steht, sowie von deren Säureadditions-Salzen und Metallsalz-Komplexen, dadurch gekennzeichnet, daß man Triazolylketone der Formel (II)

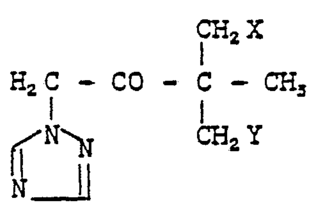

(II)

in welcher

X und Y die oben angegebene Bedeutung haben, mit Aldehyden der Formel (III)

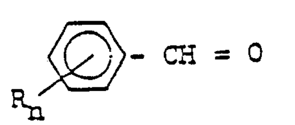

(III)

in welcher

R und n die oben angegebene Bedeutung haben,

in Gegenwart eines Lösungsmittels und in Gegenwart eines Katalysators umsetzt, gegebenenfalls anschließend die halogenierten Triazolylvinyl-keto-Derivate der Formel (Ia)

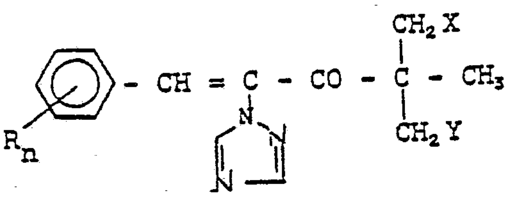

(Ia)

in welcher

X, Y, R und n die oben angegebene Bedeutung haben, in allgemein üblicher Weise reduziert, und gegebenenfalls anschließend an die so erhaltenen Verbindungen der Formel (I) eine Säure oder ein Metallsalz addiert.

6. Pflanzenwachstumsregulierende und fungizide Mittel, gekennzeichnet durch einen Gehalt an mindestens einem halogenierten Triazolylvinyl-keto- bzw. -carbinol-Derivat der Formel (I) bzw. einem Säureadditions-Salz oder Metallsalz-Komplex eines halogenierten Triazolylvinyl-keto- bzw. -carbinol-Derivates der Formel (I).

7. Verfahren zur Regulierung des Pflanzenwachstums sowie zur Bekämpfung von Pilzen, dadurch gekennzeichnet, daß man halogenierte Triazolylvinyl-keto- bzw. -carbinol-Derivate der Formel (I) bzw. deren Säureadditions-Salze oder Metallsalz-Komplexe auf die Pflanzen und/oder deren Lebensraum bzw. auf die Pilze und/oder deren Lebensraum ausbringt.

8. Verwendung von halogenierten Triazolylvinyl-keto- und -carbinol-Derivaten der Formel (I) bzw. von Säureadditions-Salzen oder Metallsalz-Komplexen von halogenierten Triazolylvinyl-keto und -carbinol-Derivaten der Formel (I) zur Regulierung des Pflanzenwachstums bzw. zur Bekämpfung von Pilzen.

9. Verfahren zur Herstellung von pflanzenwachstumsregulierenden und fungiziden Mitteln dadurch gekennzeichnet, daß man halogenierte Triazolyl-vinyl-keto- bzw. -carbinol-Derivate der Formel (I) bzw. Säureadditions-Salze oder Metallsalz-Komplexe von halogenierten Triazolylvinyl-keto bzw. -carbinol-Derivaten der Formel (I) mit Streckmitteln und/oder oberflächenaktiven Stoffen vermischt.

**Claims**

1. Halogenated triazolylvinyl-keto and -carbinol derivatives of the formula (I)

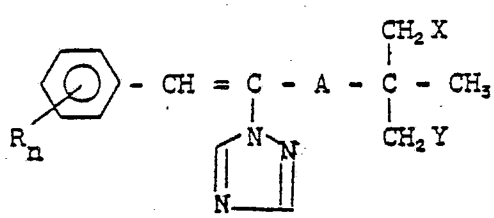

(I)

in which

A represents the keto group or the CH(OH) grouping,

X represents hydrogen, fluorine, chlorine or bromine,

Y represents fluorine, chlorine or bromine,

R represents fluorine, chlorine, bromine, alkyl with 1 to 4 carbon atoms, alkoxy with 1 to 4 carbon atoms, alkylthio with 1 to 4 carbon atoms, halogenoalkyl with 1 or 2 carbon atoms and up to 5 halogen atoms, halogenoalkoxy with 1 or 2 carbon atoms and up to 5 halogen atoms, halogenoalkylthio with 1 or 2 carbon atoms and up to 5 halogen atoms, alkylamino with 1 or 2 carbon atoms, dialkylamino with 1 or 2 carbon atoms per alkyl group, nitro, cyano, hydroxyl, alkylcarbonyloxy with 1 to 4 carbon atoms in the alkyl group, phenyl optionally substituted by fluorine, chlorine, bromine and/or alkyl with 1 or 2 carbonatoms, phenoxy optionally substituted by fluorine, chlorine, bromine and/or alkyl with 1 or 2 carbon atoms or benzyloxy optionally substituted by fluorine, chlorine, bromine and/or alkyl with 1 or 2 carbon atoms and n represents the numbers 0, 1, 2 or 3, and physiologically acceptable acid addition salts and metal salt complexes thereof.

2. Halogenated triazolylvinyl-keto and -carbinol derivatives of the formula (I), in which X represents hydrogen and Y represents fluorine or chlorine.

3. Halogenated triazolylvinyl-keto and -carbinol derivatives of the formula (I), in which X and Y are identical and represent fluorine or chlorine.

4. Halogenated triazolylvinyl-keto and -carbinol derivatives of the formula (I), in which R represents fluorine, chlorine, methyl, isopropyl, tert.-butyl, methoxy, methylthio, isopropoxy, trifluoromethyl, difluoromethyl, fluorodichloromethyl, trichloromethyl, 1,1,2-trifluoro-2-chloro-ethyl, trifluoromethoxy,

trifluoromethylthio, 1,1,2-trifluoro-2-chloro-ethoxy, and -ethylthio, dimethylamino, nitro, cyano, hydroxyl, acetoxy, tert.-butylcarbonyloxy, and benzyloxy, phenoxy and phenyl optionally mono- or di-substituted by identical or different substituents selected from fluorine, chlorine and/or methyl.

5. Process for the preparation of halogenated triazolylvinyl-keto and -carbinol derivatives of the formula (I)

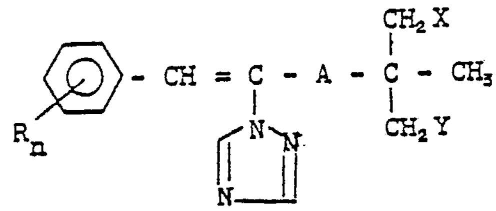

(I)

in which

A represents the keto group of the CH(OH) grouping,

X represents hydrogen, fluorine, chlorine or bromine,

Y represents fluorine, chlorine or bromine,

R represents fluorine, chlorine, bromine, alkyl with 1 to 4 carbon atoms, alkoxy with 1 to 4 carbon atoms, alkylthio with 1 to 4 carbon atoms, halogenoalkyl with 1 or 2 carbon atoms and up to 5 halogen atoms, halogenoalkoxy with 1 or 2 carbon atoms and up to 5 halogen atoms, halogenoalkylthio with 1 or 2 carbons atoms and up to 5 halogen alkylamino with 1 or 2 carbon atoms, dialkylamino with 1 or 2 carbon atoms per alkyl group, nitro, cyano, hydroxyl, alkylcarbonyloxy with 1 to 4 carbon atoms in the alkyl group, phenyl optionally substituted by fluorine, chlorine, bromine and/or alkyl with 1 or 2 carbon atoms, phenoxy optionally substituted by fluorine, chlorine, bromine and/or alkyl with 1 or 2 carbon atoms or benzyloxy optionally substituted by fluorine, chlorine, bromine and/or alkyl with 1 or 2 carbon atoms and n represents the numbers 0, 1, 2, or 3, and acid addition salts and metal salts complexes thereof, characterised in that triazolyl-ketones of the formula (II)

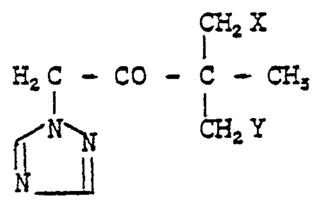

(II)

in which

X and Y have the abovementioned meaning, are reacted with aldehydes of the formula (III)

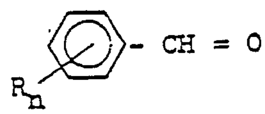

(III)

in which

R and n have the abovementioned meaning, in the presence of a solvent and in the presence of a catalyst, and, if desired, the halogenated triazolylvinyl-keto derivatives of the formula (Ia)

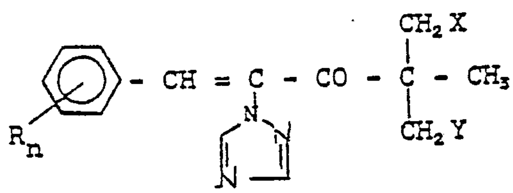

(Ia)

in which

X, Y, R and n have the abovementioned meaning, are then reduced in a generally customary manner, and, if desired, an acid or a metal salt is then added on to the compounds of the formula (I) thus obtained.

6. Plant-growth-regulating and fungicidal agents, characterised in that they contain at least one halogenated triazolylvinyl-keto or -carbinol derivative of the formula (I) or at least one acid addition salt or metal salt complex of a halogenated triazolylvinyl-keto or -carbinol derivative of the formula (I).

7. Process for regulating plant growth and for combating fungi, characterised in that halogenated triazolylvinyl-keto or -carbinol derivatives of the formula (I) or acid addition salts or metal salt complexes thereof are applied to the plants and/or their environment or to the fungi and/or their environment.

8. Use of halogenated triazolylvinyl-keto and -carbinol derivatives of the formula (I) or acid addition salts or metal salt complexes of halogenated triazolylvinyl-keto and -carbinol derivatives of the formula (I) for regulating plant growth or for combating fungi.

9. Process for the preparation of plant-growth-regulating and fungicidal agents, characterised in that halogenated triazolylvinyl-keto or -carbinol derivatives of the formula (I) or acid addition salts or metal salt complexes of halogenated triazolylvinyl-keto or -carbinol derivatives of the formula (I) are mixed with extenders and/or surface-active substances.

**Revendications**

1. Dérivés triazolylvinylcétoniques et carbinoliques halogénés de formule (I)

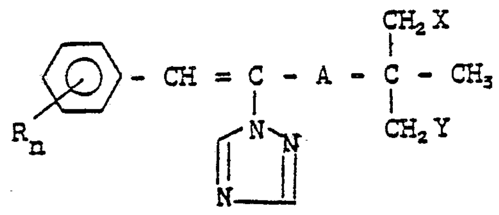

(I)

dans laquelle

A représente le groupe céto ou le groupement CH(OH),

X est l'hydrogène, le fluor, le chlore ou le brome,

Y est le fluor, le chlore ou le brome,

R représente le fluor, le chlore, le brome, un groupe alkyle ayant 1 à 4 atomes de carbone, alkoxy ayant 1 à 4 atomes de carbone, alkylthio ayant 1 à 4 atomes de carbone, halogénalkyle ayant 1 ou 2 atomes de carbone et jusqu'à 5 atomes d'halogènes, halogénalkoxy ayant 1 ou 2 atomes de carbone et jusqu'à 5 atomes d'halogènes, halogénalkylthio ayant 1 ou 2 atomes de carbone et jusqu'à 5 atomes d'halogènes, alkylamino ayant 1 ou 2 atomes de carbone, dialkylamino ayant 1 ou 2 atomes de carbone par groupe alkyle, nitro, cyano, hydroxy, alkylcarbonyloxy ayant 1 à 4 atomes de carbone dans le groupe alkyle, un groupe phényle éventuellement substitué par du fluor, du chlore, du brome et/ou un radical alkyle ayant 1 ou 2 atomes de carbone, un groupe phénoxy éventuellement substitué par du fluor, du chlore, du brome et/ou un radical alkyle ayant 1 ou 2 atomes de carbone, et un groupe benzyloxy éventuellement substitué par du fluor, du chlore, du brome et/ou un radical alkyle ayant 1 ou 2 atomes de carbone et n représente les nombres 0, 1, 2 ou 3, ainsi que leurs sels d'addition d'acides et leurs complexes de sels métalliques acceptables du point de vue physiologique.

2. Dérivés triazolylvinylcétoniques et -carbinoliques halogénés de formule (I), dans lesquels X représente l'hydrogène et Y représente le fluor ou le chlore.

3. Dérivés triazolylvinylcétoniques et -carbinoliques halogénés de formule (I), dans lesquels X et Y sont égaux et représentent du fluor ou du chlore.

4. Dérivés triazolylvinylcétoniques et -carbinoliques halogénés de formule (I), dans lesquels R représente le fluor, le chlore, un groupe méthyle, isopropyle, tertio-butyle, méthoxy, méthylthio, isopropoxy, trifluorométhyle, difluorométhyle, fluorodichlorométhyle, trichlorométhyle, 1,1,2-trifluoro-2-chloréthyle, trifluorométhoxy, trifluorométhylthio, 1,1,2-trifluoro-2-chloréthoxy et -éthylthio, diméthylamino, nitro, cyano, hydroxy, acétoxy, tertio-butylcarbonyloxy, ainsi qu'un groupe phényle, phénoxy et benzyloxy portant éventuellement un ou deux substituants fluor, chlore et/ou méthyle égaux ou différents.

5. Procédé de production de dérivés triazolylvinylcétoniques et -carbinoliques halogénés de formule (I)

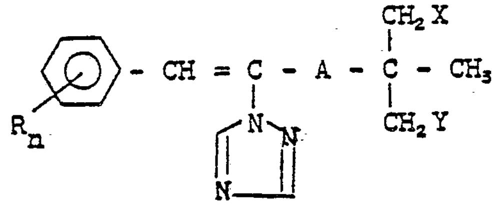

(I)

dans laquelle

A représente le groupe céto ou le groupement CH(OH),

X est l'hydrogène, le fluor, le chlore ou le brome,

Y est le fluor, le chlore ou le brome,

R représente le fluor, le chlore, le brome, un groupe alkyle ayant 1 à 4 atomes de carbone, alkoxy ayant 1 à 4 atomes de carbone, alkylthio ayant 1 à 4 atomes de carbone, halogénalkyle ayant 1 ou 2

atomes de carbone et jusqu'à 5 atomes d'halogènes, halogénalkoxy ayant 1 ou 2 atomes de carbone et jusqu'à 5 atomes d'halogènes, halogénalkylthio ayant 1 ou 2 atomes de carbone et jusqu'à 5 atomes d'halogènes, alkylamino ayant 1 ou 2 atomes de carbone dialkylamino ayant 1 ou 2 atomes de carbone par groupe alkyle, nitro, cyano, hydroxy, alkylcarbonyloxy ayant 1 à 4 atomes de carbone dans le groupe alkyle, un groupe phényle éventuellement substitué par du fluor, du chlore, du brome et/ou un radical alkyle ayant 1 ou 2 atomes de carbone, un groupe phénoxy éventuellement substitué par du fluor, du chlore, du brome et/ou un radical alkyle ayant 1 ou 2 atomes de carbone, et un groupe benzyloxy éventuellement substitué par du fluor, du chlore, du brome et/ou un radical alkyle ayant 1 ou 2 atomes de carbone et

n représente les nombres 0, 1, 2 ou 3, ainsi que de leurs sels d'addition d'acides et de leurs complexes de sels métalliques, caractérisé en ce qu'on fait réagir des triazolylcétones de formule (II)

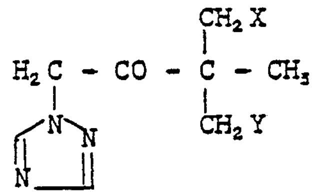

(II)

dans laquelle

X et Y ont la définition indiquée ci-dessus, avec des aldéhydes de formule (III)

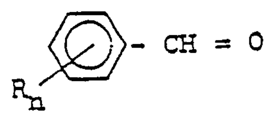

(III)

dans laquelle

R et n ont la définition indiquée ci-dessus, en présence d'un solvant et en présence d'un catalyseur, puis on réduit éventuellement les dérivés triazolylvinylcétoniques halogénés de formule (Ia)

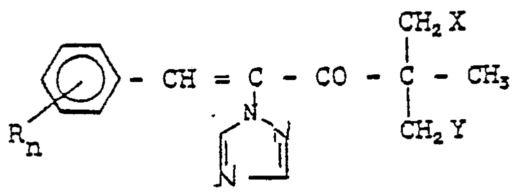

(Ia)

dans laquelle

X, Y, R et n ont la définition indiquée ci-dessus, d'une manière généralement classique et le cas échéant on additionne sur les composés de formule (I) ainsi obtenus un acide ou un sel métallique.

6. Compositions influençant la croisance des plantes et fongicides, caractérisées par une teneur en au moins un dérivé triazolylvinylcétonique ou -carbinolique halogéné de formule (I) ou en un sel d'addition d'acide ou en un complexe de sel métallique d'un dérivé triazolylvinylcétonique ou -carbinolique halogéné de formule (I).

7. Procédé pour influencer la croissance de végétaux ainsi que pour combattre des champignons, caractérisé en ce qu'on applique des dérivés triazolylvinylcétoniques ou -carbinoliques halogénés de formule (I) ou leurs sels d'addition d'acides ou leurs complexes de sels métalliques sur les plantes et/ou sur leur milieu ou respectivement sur les champignons et/ou sur leur milieu.

8. Utilisation de dérivés triazolylvinylcétoniques ou -carbinoliques halogénés de formule (I) ou respectivement de sels d'addition d'acides ou de complexes de sels métalliques de dérivés triazolylvinylcétoniques et -carbinoliques halogénés de formule (I) pour influencer la croissance des plantes et respectivement pour combattre des champignons.

9. Procédé de préparation de compositions influençant la croissance des plantes et fongicides, caractérisé en ce qu'on mélange des dérivés triazolylvinylcétoniques ou -carbinoliques halogénés de formule (I) ou respectivement des sels d'addition d'acides ou des complexes de sels métalliques de dérivés triazolylvinylcétoniques ou -carbinoliques halogénés de formule (I) avec des diluants et/ou des agents tensio-actifs.